(19) Europäisches Patentamt
European Patent Office
Office européen des brevets

(11) Veröffentlichungsnummer: **0 427 190 A2**

# (12) EUROPÄISCHE PATENTANMELDUNG

(21) Anmeldenummer: **90121190.4**

(22) Anmeldetag: **06.11.90**

(51) Int. Cl.⁵: **A61K 31/725**, C08L 5/14, //(C08L5/14,5:08),(A61K31/725, 31:73,37:02)

(30) Priorität: **09.11.89 DE 3937283**

(43) Veröffentlichungstag der Anmeldung: **15.05.91 Patentblatt 91/20**

(84) Benannte Vertragsstaaten: **AT BE CH DE DK ES FR GB GR IT LI LU NL SE**

(71) Anmelder: **HOECHST AKTIENGESELLSCHAFT**
**Postfach 80 03 20**
**W-6230 Frankfurt am Main 80(DE)**

(72) Erfinder: **Krone, Volker, Dr.**
**Friedrich-Ebert-Strasse 26**
**W-6093 Flörsheim(DE)**
Erfinder: **Magerstädt, Michael, Dr.**
**Rheingaustrasse 19**
**W-6238 Hofheim am Taunus(DE)**
Erfinder: **Schrinner, Elmar, Dr.**
**Hedwigstrasse 2**
**W-6200 Wiesbaden(DE)**
Erfinder: **Walch, Axel, Dr.**
**Hans-Sachs-Strasse 5**
**W-6000 Frankfurt am Main(DE)**

## (54) Polyelektrolytkomplexe zur Behandlung und Prophylaxe von Viruserkrankungen sowie Verfahren zu deren Herstellung.

(57) Polyelektrolytkomplexe bestehend aus einer Polysäure und einer Polybase eignen sich zur Behandlung und Prophylaxe von Viruserkrankungen.

EP 0 427 190 A2

Xerox Copy Centre

# POLYELEKTROLYTKOMPLEXE ZUR BEHANDLUNG UND PROPHYLAXE VON VIRUSERKRANKUNGEN, NEUE POLYELEKTROLYTKOMPLEXE SOWIE VERFAHREN ZU DEREN HERSTELLUNG

Die vorliegende Erfindung betrifft Polyelektrolytkomplexe zur Behandlung und Prophylaxe von Viruserkrankungen, neue Polyelektrolytkomplexe sowie Verfahren zu deren Herstellung.

Gegen virale Erkrankungen in Mensch und Tier sind bisher nur wenige Behandlungsmöglichkeiten bekannt. Insbesondere die Infektion mit dem menschlichen Immundefizienz-Virus (HIV), welches in engen kausalen Zusammenhang mit der erworbenen Immunschwäche AIDS gebracht wird, kann bisher nur in begrenztem Maße behandelt werden.

In neueren Arbeiten wurde gezeigt, daß neben dem bisher auf dem Markt befindlichen, mit starken Nebenwirkungen behafteten Präparat Azidothymidin (AZT), auch sulfatierte Oligo- und Polysaccharide eine Hemmwirkung gegen das HIV zeigen. Diese äußert sich u.A. in vivo in einer Inhibition der Reversen Transcriptase des HIV sowie Hemmung der Syncytienbildung bei infizierten kultivierten menschlichen T-Lymphocyten. Anhaltspunkte auf eine Wirksamkeit im Menschen bestehen ebenfalls. Insbesondere Xylanpolysulfat und dessen sulfatierte Alkylesterderivate zeigen besonders starke Wirkung. Bezüglich dieser Verbindungen resultiert ein besonderes Problem daraus, daß alle sulfatierten Oligo-und Polysaccharide eine mehr oder weniger starke antikoagulatorische Wirkung zeigen. Dieser Umstand allein ware auch bei einer Dauertherapie nicht unbedingt als schwere Nebenwirkung anzusehen. Da aber die biologischen Halbwertszeiten dieser Verbindungen im Bereich einiger Stunden liegen, müßten bei intravenöser, intramuskulärer oder subcutaner Injektion jeweils eine bis mehrere Injektionen täglich verabreicht werden. Durch die verringerte Koagulationsfähigkeit können hierbei an der Einstichstelle Blutungen entstehen, was bei derart häufiger Behandlung ernste Folgen haben kann.

Da die orale Resorbierbarkeit von sulfatierten Polysacchariden teilweise nicht befriedigend ist, besteht das Bedürfnis nach besseren, insbesondere oral resorbierbaren Chemotherapeutika zur Bekämpfung des HIV.

Überraschenderweise wurde nun gefunden, daß Polyelektrolytkomplexe aus mindestens einer Polysäure und mindestens einer Polybase, wobei eine der Komponenten antivirale Wirksamkeit besitzt, eine gute antivirale Wirksamkeit aufweisen und gleichzeitig oral gut resorbiert werden. Diese Eigenschaften der genannten Polyelektrolyten waren nicht zu erwarten, insbesondere weil die in den Polysäuren und Polybasen zahlreich vorhandenen Ladungen eher eine schlechte orale Resorbierbarkeit vermuten lassen.

Erfindungsgegenstand sind demzufolge Polyelektrolykomplexe aus mindestens einer Polysäure und mindestens einer Polybase zur Behandlung und Prophylaxe von Viruserkrankungen. Erfindungsgemäß bevorzugte Polysäuren sind teilweise oder vollständig sulfatierte unsubstituierte oder substituierte Oligo- oder Polysaccharide.

Bevorzugte unsubstituierte Polysaccharide sind z.B. in der DE-OS 37 25 554 und der EP 0270317 beschrieben. Ein besonders bevorzugten sulfatiertes Polysaccharid ist Xylanpolysulfat.

Erfindungsgemäß bevorzugte substituierte Polysaccharide (wie in der deutschen Patentanmeldung P 39 21 761 beschrieben) sind Polysaccharide, die linear oder verzweigt sein können, bestehend aus einheitlichen oder unterschiedlichen natürlichen oder synthetischen Monomeren, die pro Monomereinheit auch eine substituierte oder unsubstituierte Aminogruppe enthalten können und deren OH-Gruppen durchschnittlich zwischen 5 und 80 % substituiert sind mit einer Gruppe der Formel -X-B-Y, wobei X eine Oxygruppe oder eine Gruppe der Formel I bedeutet

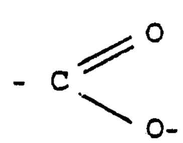

(I)

deren Kohlenstoffatom an B gebunden ist,

B einen nicht-aromatischen Kohlenwasserstoffrest mit 2 bis 30 C-Atomen bedeutet, in dessen Alkylkette bis zu 3 Methyleneinheiten durch Oxygruppen ersetzt sein können, in der bis zu drei C-C-Doppelbindungen vorliegen können und der substituiert sein kann mit bis zu drei $C_1$-$C_4$-Alkylresten und

Y - falls X eine Oxygruppe ist - Wasserstoff, COOR, $OSO_3R^1$ sein kann, worin R ein physiologisch verträgliches ein-oder zweiwertiges Kation ist, oder einen Kohlenwasserstoffrest mit bis zu 20 C-Atomen oder einen Mono- oder Bisetherrest mit 3 bis 10 C-Atomen darstellt, $R^1$ ein physiologisch verträgliches

Kation darstellt oder

Y - falls X ein Rest der Formel I ist - Wasserstoff oder COOR darstellt, worin R die obengenannten Bedeutungen hat

und wobei die OH-Gruppen der obengenannten Polysaccharide zwischen 10 und 95 % substituiert sind mit einer Gruppe der Formel $OSO_3M$, wobei M ein physiologisch verträgliches Kation darstellt und zwischen 0 und 40 % der OH-Gruppen unsubstituiert sind.

Zum Erfindungsgegenstand gehören weiterhin die bisher noch nicht beschriebenen Polyelektrolytkomplexe bestehend aus Xylansulfat, dessen lipophilen Derivaten oder lipophilen Derivaten des Dextransulfats und einer Polybase oder bestehend aus Dextransulfat und Polylysin einem Lysinester mit einer Estergruppe mit 10-20 C-Atomen, Chitosan oder Poly-(2-N,N-Dimethyl-aminoethyl)-D,L-aspartamid.

Der Sulfatierungsgrad der sulfatierten Polysaccharide kann einen weiten Bereich überstreichen. Bevorzugt ist ein Sulfatierungsgrad von mehr als 10 %, besonders bevorzugt von mehr als 50 %, insbesondere zwischen 90 und 100 %.

Das mittlere Molekulargewicht der unsubstituierten und substituierten Polysaccharide sollte zwischen 500 und 80000 D, bevorzugt zwischen 1000 und 40000 D, insbesondere zwischen 4000 und 15000 D liegen.

Die erfindungsgemäßen Polyelektrolytkomplexe können mit Hilfe unterschiedlicher Polybasen gebildet werden. U.a. können Proteine als Polybasen eingesetzt werden, wie z.B. Hämoglobin. Weiterhin sind z.B. Polylysin, Lysinalkylester, Chitosan Poly-(2-N,N-Dimethyl-aminoethyl)-D,L-aspartamid, Aminosäuren, Polyaminosäuren, aminierte Oligo- und Polysaccharide (z.B. aminierte Dextrane),Poly-[$\alpha$,$\beta$-(spermidinyl)-D,L-aspartamid], Gelatine und Derivate (z.B. Polygeline®, Behringwerke AG, Marburg), quarternäre Ammoniumverbindungen (z.B. Luviquat®, BASF AG, Ludwigshafen oder Mirapol A15®, Miranal Chem. Co., Inc. South Brunswick, New Jersey, $C_{12}$-Alkylsternamin, $\alpha$,$\omega$-Bis-(N-propylamino)polyethylenglycol sowie aminierte Polyethylenglycolderivate, geeignet. Weiterhin können als Polybasen kovalente Verbindungen aus OH-Gruppen-haltigen Polymeren (z.B. Dextran) und Stickstoffkronenethern sowie deren Metallkomplexe eingesetzt werden.

Im Falle des Polylysins sollte die Polybase vorzugsweise ein Molekulargewicht von 1000 bis 200000, besonders bevorzugt von 2000 bis 50000 haben.

Ein Beispiel eines erfindungsgemäß besonders geeigneten Polyelektrolytkomplexes ist ein Komplex aus Xylanpolysulfat und Hämoglobin.

Weiterhin ist es möglich, die erfindungsgemäßen Polyelektrolytkomplexe durch Variation des Substituenten der Polysäuren und Polybasen in Bezug auf Ihre Resorptionsfähigkeit zu optimieren. So ist es z.B. möglich, die Polysäuren mit unterschiedlichen Substituenten, wie oben bereits erläutert, auszustatten. Die Polybasen können ebenfalls durch Substitutionsreaktionen verändert werden. Besonders geeignete Substituenten für die Polybasen sind hydrophobisierender Natur, wie z.B. Alkylketten, insbesondere verzweigte oder unverzweigte Alkylketten mit 10-20 C-Atomen, wie z.B. Lysinoctadecylester oder Cetyltrimethylammonium-bromid. Andere geeignete Substituenten sind z.B. Steroidreste, insbesondere Cholesterol über die Alkoholgruppe gebunden bzw. der Cholesterylester der Bernsteinsäure oder analoge Steroide.

Darüber hinaus ist es möglich, maßgeschneiderte Polylektrolykomplexe durch die Umsetzung einer Polysäure oder einer Mischung von Polysäuren mit einer Polybase oder einer Mischung von Polybasen herzustellen.

Weiterhin gehört zum Erfindungsgegenstand ein Verfahren zur Herstellung von Polyelektrolytkomplexen, das dadurch gekennzeichnet ist, das entweder a) die Polysäure oder b) die Polybase vorzugsweise in wäßriger Lösung vorgelegt wird und a) die Polybase oder b) die Polysäure bei geeigneter Temperatur und bei geeigneten pH-Wert vorzugsweise in wäßriger Lösung zugetropft wird.

Die jeweilige Konzentration der Polysäure und der Polybase in der wäßrigen Lösung, die Temperatur und der pH-Wert können in weiten Bereichen variiert werden; die jeweils optimalen Parameter sind empirisch für jede Kombination zu ermitteln. Repräsentative Parameter sind in den Ausführungsbeispielen angegeben.

Die Mengenverhältnisse, in denen die Polysäuren und Polybasen miteinander zur Reaktion gebracht werden, können einen weiten Bereich überstreichen. Bevorzugt ist ein Gewichtsverhältnis von 1:10 bis 10:1, besonders bevorzugt von 1:3 bis 3:1, insbesondere von ca. 1:1. In vielen Fällen kann auch ein ungefähr äquimolares Verhältnis der beiden Komponenten vorteilhaft sein.

Zum Erfindungsgegenstand gehört weiterhin die Verwendung von Polyelektrolytkomplexen zur Behandlung oder Prophylaxe von Viruserkrankungen, sowie Arzneimittel mit einem Gehalt an Polyelektrolytkomplexen.

Zur Behandlung bzw. zur Prophylaxe von Erkrankungen, die durch Viren verursacht werden, können die

erfindungsgemäßen Polyelektrolytkomplexe auf unterschiedliche Weise verabreicht werden. Z.B. können sie intravenös, intramuskulär, intraperitoneal, subkutan, als Dauertropfinfusion oder oral verabreicht werden. Die orale Verabreichung ist bevorzugt.

Die Herstellung der erfindungsgemäßen Arzneimittel erfolgt, indem mindestens ein Polyelektrolytkomplex, gegebenenfalls mit weiteren Zusatz- und/oder Hilfsstoffen, in eine geeignete Darreichungsform gebracht wird. Die Zusatz- oder Hilfsstoffe stammen aus der Gruppe der Trägermittel, Konservierungsstoffe und anderer üblicher Hilfsstoffe. Z.B. können für orale Darreichungsformen Hilfsstoffe wie Stärke, z.B. Kartoffel-, Mais- oder Weizenstärke, Zellulose bzw. deren Derivate, insbesondere mikrokristalline Cellulose, Siliziumdioxid, verschiedene Zucker wie Milchzucker, Magnesiumcarbonat und/oder Calciumphosphate verwendet werden. Weiterhin kann es vorteilhaft sein, den oralen Darreichungsformen Hilfsstoffe zuzusetzen, die die Verträglichkeit des Medikamentes verbessern, wie z.B. Schleimbildner und Harze. Zwecks besserer Verträglichkeit kann das Medikament auch in Form von magensaftunlöslichen Kapseln verabreicht werden. Darüber hinaus kann es vorteilhaft sein, der Darreichungsform ein Retardierungsmittel zuzusetzen, gegebenenfalls in Form von permeablen Membranen, wie z.B. solche auf Cellulose- oder Polystyrolharzbasis, oder auf der Basis von Ionenaustauschern.

Die anzuwendende Dosierung der erfindungsgemäßen Polyelektrolytkomplexe ist abhängig von verschiedenen Faktoren wie Darreichungsform des Medikaments und Zustand und Gewicht des Patienten. Eine Tagesdosis von ca 3000 mg an Polyelektrolykomplex sollte jedoch nur kurzzeitig überschritten werden. Bevorzugt sind ca. 200 bis 1500 mg Polyelektrolytkomplex als Tagesdosis. Darüber hinaus ist es zweckmäßig, für einzelne Polyelektrolytkomplexe sowohl Zusammensetzung als auch Dosierung in Versuchen zu testen.

Die erfindungsgemäßen Polyelektrolytkomplexe weisen eine Wirkung gegen Viren insbesondere auch bei der oralen Verabreichung auf. Sie können sowohl zur Behandlung als auch zur Prophylaxe von Viruserkrankungen verwendet werden.

Besondere Bedeutung besitzen die erfindungsgemäßen Polyelektrolytkomplexe für die Bekämpfung von Retroviren, insbesondere bei der Bekämpfung der verschiedenen Arten des HIV.

Durch die nachfolgenden Ausführungsbeispiele soll die vorliegende Erfindung näher erläutert werden.

## Beispiele

### 1. Darstellung des Polyelektrolytkomplexes aus Xylanpolysulfat und Poly-L-Lysin:

Eine 0,1 % Lösung von 5 g Xylanpolysulfat (Hersteller: Fa. BENE-Chemie, München), in der Natriumsalzform (gilt auch für alle folgenden Erwähnungen von Xylanpolysulfat) entsprechend 14,8 mmol Monomereinheiten, in Wasser wird mit einer 0,1 % wäßrigen Lösung von 29,6 mmol Monomereneinheiten Poly-L-Lysin (mittleres Molekulargewicht 3500 D) versetzt und gerührt. Der Niederschlag wird über eine Glasfritte abfiltriert, mit $H_2O$ gewaschen und gefriergetrocknet. Das Produkt wird durch Elementaranalyse und pH-Bestimmung einer wäßrigen Lösung charakterisiert. Ausbeute: 8,2 g.

### 2.a) Darstellung von Poly-(2-N,N-dimethylaminoethyl)-D,L-aspartamid (PDAA)

22,28 g Polysuccinimid (mittleres Molekulargewicht ca. 4700, über Viskosimetrie bestimmt) werden in 92 ml DMF gelöst, dann werden 4,59 g Hydroxypyridin und 45,94 ml 2-Dimethylaminoethylamin unter Eiskühlung und Rühren dazugegeben. Nach Rühren über 15 h wird durch Eingießen in wasserfreies Aceton gefällt, über eine Glasfritte abfiltriert und mit Aceton der Rückstand neutralgewaschen. Das Produkt wird im Vakuumexsiccator über $P_2O_5$ getrocknet. Charakterisierung erfolgt durch Elementaranalyse, NMR und IR-Spektroskopie; das Molekulargewicht wird viskosimetrisch bestimmt. Ausbeute: 51 g.

### b) Darstellung des Polyelektrolykomplexes aus Xylanpolysulfat und Poly-(2-N,N-dimethylaminoethyl)-D,L-aspartamid (PDAA)

Je eine 0,1 % wäßrige Lösung von Xylanpolysulfat und PDAA werden in gleichen Mengen (jeweils 1 l) zusammengegeben.

Nach weiterem Rühren (1 h) wird der Niederschlag über eine Glasfritte abfiltriert, mit $H_2O$ gewaschen

und gefriergetrocknet. Ausbeute: 1,8 g. Charakterisierung über Elementaranalyse und pH-Bestimmung einer wäßrigen Lösung.

**3. Darstellung des Polyelektrolytkomplexes aus Xylanpolysulfat und Chitosan**

5 g Xylanpolysulfat (14,8 mmol Monomereneinheiten) werden in der minimal benötigten Menge Wasser gelöst und über einen Ionenaustauscher in der H+ Form gegeben. Das saure Eluat wird dann bis zur Neutralität mit einer 0,1 % wäßrigen Lösung von Chitosan (Fa. Protan) versetzt. Dabei fällt der Polyelektrolytkomplex aus. Nach 1 h Rühren wird das Produkt wie in Beispiel 2 beschrieben aufgearbeitet und charakterisiert. Ausbeute: 8,7 g.

**4. Darstellung des Polyelektrolytkomplexes aus Dextransulfat und Chitosan**

15 mmol Monomereneinheiten Dextransulfat (hergestellt gemäß der deutschen Patentanmeldung P 39 21 769.2) werden in der minimal benötigten Menge Wasser gelöst und über einen Ionenaustauscher in der H+ Form gegeben. Das saure Eluat wird dann bis zur Neutralität mit einer 0,1 % wäßrigen Lösung von Chitosan (Hersteller: Fa. Protan & Fagertun, Drammen, Norwegen) versetzt. Dabei fällt der Polyelektrolytkomplex aus. Nach 1 h Rühren wird das Produkt wie in Beispiel 2 beschrieben aufgearbeitet und charakterisiert. Ausbeute: 8,2 g.

**5. Darstellung des Polyelektrolytkomplexes aus partiell Palmitoyl-substituiertem Xylanpolysulfat und Poly-L-Lysin**

Das partiell Palmitoyl-substituierte Xylanpolysulfat wird gemäß der deutschen Patentanmeldung P 39 21 761.2 dargestellt. Eine einprozentige Lösung von Poly-L-lysin Hydrobromid, Molekulargewicht 3900 (Hersteller: Fa. Sigma Chemie, München) in Wasser, mit HCl auf pH 4 eingestellt, wird bei Raumtemperatur vorgelegt. Dazu tropft man unter Rühren eine einprozentige Lösung des Xylanpolysulfatderivats in Wasser, die ebenfalls mit HCl auf pH 4 eingestellt wurde. Dabei fällt der Polyelektrolytkomplex aus. Der Niederschlag wird durch Zentrifugation abgetrennt und gefriergetrocknet. Das trockene Produkt wird mit Wasser gewaschen und erneut zentrifugiert. Dann wird der Niederschlag abgetrennt und wieder gefriergetrocknet. Das Produkt wird wie in Beispiel 2 beschrieben charakterisiert. Ausbeute: ca. 50 % d.Th.

**6. a) Darstellung von L-Lysinoctadecylester**

333 mMol Octadecanol (Hersteller: Fa Riedel-de Haen, Seelze) werden bei 100° C geschmolzen und mit 164 mMol L-Lysin Hydrochlorid (Hersteller: Fa. Sigma, München) versetzt. Dann werden zu der entstehenden Suspension innerhalb 10 Minuten 272 mMol Methansulfonsäure getropft. Die Schmelze wird 2 h bei 115° C gerührt, dann in 1 l stark gerührten Diethylether bei -70° C (unter $N_2$) gegossen. Nach Erwärmen auf Raumtemperatur wird 1 h gerührt, der Niederschlag über eine G4-Glasfritte abgetrennt und mehrmals mit Diethylether gewaschen. Der Rückstand wird unter Rühren in eine Mischung aus 600 ml $H_2O$ und 100 ml $CH_3OH$ gegeben. Bei 50° C wird 10 Minuten gerührt und dann auf Raumtemperatur abgekühlt. Ca. 150 ml 2 m NaOH werden über 20 Minuten zugetropft, bis pH 12 erreicht ist. Der entstehende dicke Brei wird auf 10° C gekühlt, einmal mit 500 ml Diethylether und dann zweimal mit je 200 ml Diethylether extrahiert. Die Etherextrakte werden über $Na_2SO_4$ getrocknet und auf -10° C gekühlt. Man leitet HCl-Gas ein bis zur Sättigung. Der Niederschlag wird über eine G2-Glasfritte abgetrennt, mehrmals mit Diethylether gewaschen und im Vakuum getrocknet. Ausbeute: 75 g (95 % d.Th.). Charakterisierung über $^1$H-NMR und Dünnschichtchromatographie in $CH_3OH$: 25 % Ammoniak 9:1 auf Kieselgel, $R_f$ = 0,4.

**b) Darstellung des Polyelektrolytkomplexes aus Xylanpolysulfat und L-Lysinoctadecylester**

Es wird analog zu Beispiel 5 verfahren, wobei das Poly-L-Lysin durch L-Lysinoctadecylester ersetzt wird.

**Wirksamkeitstest**

Wirksamkeit von Polyelektrolytkomplexen sulfatierter Polysaccharide gegen Retrovirusinfektionen in der Maus

Da für die HIV-Infektion des Menschen kein geeignetes Infektionsmodell bei Labortieren existiert, muß bei der Prüfung von Chemotherapeutika auf Infektionen mit anderen Retroviren zurückgegriffen werden. Im vorliegenden Fall wird die Infektion der Maus mit dem Friend-Leukämie-Virus gewählt. Dazu werden normale Labormäuse (NMRI = Naval Medical Research Institute) durch intravenöse Infektion mit Friend-Leukämie- Virus enthaltendem Mäuseserum infiziert. Bei den unbehandelten Kontrolltieren entwickelt sich als Symptom der Infektion innerhalb von 2 Wochen eine deutliche Vergrößerung von Milz und Leber. Die Behandlung erfolgt über 10 Tage, beginnend 48 Stunden nach der Infektion oder über 16 Tage, beginnend 4 Tage vor der Infektion. Am 14. Versuchstag werden die Tiere durch Luxation der Halswirbel getötet und geöffnet. Die Milz wird entnommen und gewogen. Als Meßparameter der therapeutischen Wirksamkeit wird das Milzgewicht der behandelten Tiere mit dem der unbehandelten Infektionskontrolle in Relation gesetzt.

Während bei uninfizierten ausgewachsenen Labormäusen (20-24 g Körpergewicht) die Milz etwa 1 % des Körpergewichtes oder weniger wiegt, erreicht bei infizierten Tieren die Milz am Ende des Versuchs etwa 10 % des Körpergewichts.

Die Tabelle 1 zeigt die Ergebnisse der Wirksamkeitstests bei der Friend-Leukämie-Virusinfektion in der Maus bei intraperitonealer oder oraler Verabreichung der erfindungsgemäßen Verbindungen. Als Kontrolle dient eine Gruppe von Mäusen, die unbehandelt bleiben.

Der Behandlungserfolg ist erkennbar an der Verringerung der Milzschwellung.

Die untersuchten Verbindungen erweisen sich bei zehnmaliger oraler Verabreichung von 2000 mg/kg als wirksamer als Xylanpolysulfat 6000, dessen Wirksamkeit gegen HIV in vitro und in retroviralen Mäusemodell bekannt ist. (Biesert et al. Inhibition of HIV and virus replication by sulphates polyxylan: HOE/BAY 946, a new antiviral compound. AIDS 2, 449 - 457 (1988). Baba et al. Pentosan polysulfate, a sulfated oligosaccharide, is a potent and selective anti-HIV agent in vitro. Antiviral Res. 9, 335 -343 (1988))

Die Reduktion des Milzgewichtes gegenüber der infizierten Kontrolle ist statistisch signifikant ($p < 0,05$ Student's-T-Test).

**Wirksamkeit von Polyelektrolytkomplexen sulfatierter Polysaccharide gegen Retrovirusinfektionen in der Maus**

Tabelle 1

| Präparat | Dosierung (mg/kg) | rel. Milzgewicht (%) | n | p |
|---|---|---|---|---|
| Kontrolle | | 8,66 ± 3,16 | (10) | 1,00 |
| 1. Xylanpolysulfat | 10 x 2000 p.o. | 6,36 ± 2,48 | (10) | 0,04 |
| 2. Xylanpolysulfat-PDAA-Polyelektrolytkomplex nach Beispiel 2 | 10 x 2000 p.o. | 5,35 ± 1,38 | (10) | 0,04 |
| 3. Xylanpolysulfat-Poly-L-Lysin-Polyelektrolytkomplex nach Beispiel 1 | 10 x 2000 p.o. | 5,24 ± 1,63 | (10) | 0,04 |
| 4. Xylanpolysulfat-Chitosan-Polyelektrolytkomplex nach Beispiel 3 | 16 x 1000 p.o. | 6,37 ± 3,23 | (5) | 0,04 |
| | 16 x 2000 p.o. | 5,90 ± 1,51 | (5) | 0,005 |

Der Behandlungserfolg war erkennbar an der Verringerung der Milzschwellung.

Die untersuchten Verbindungen erwiesen sich bei zehnmaliger oraler Verabreichung von 2000 mg/kg

als wirksamer als Xylanpolysulfat 6000, dessen Wirksamkeit gegen HIV in vitro (1/2) und in retroviralen Mäusemodell (1) bekannt ist. Die Reduktion des Milzgewichtes gegenüber der infizierten Kontrolle war statistisch signifikant ($p< 0,05$ Student's-T-Test).

## Ansprüche

1. Polyelektrolytkomplexe aus mindestens einer Polysäure und mindestens einer Polybase zur Behandlung und Prophylaxe von Viruserkrankungen.
2. Polyelektrolytkomplexe gemäß Anspruch 1, dadurch gekennzeichnet, daß die Polysäure ein teilweise oder vollständig sulfatiertes, unsubstituiertes oder substituiertes Oligo- oder Polysaccharid ist.
3. Polyelektrolytkomplexe gemäß Anspruch 1 oder 2, dadurch gekennzeichnet, daß die Polysäure ein Oligo- oder Polysaccharid ist, deren OH-Gruppen teilweise verethert oder verestert sind.
4. Polyelektrolytkomplexe gemäß einem oder mehreren der Ansprüche 1 bis 3, dadurch gekennzeichnet, daß die Polybase ein Protein ist.
5. Polyelektrolytkomplexe gemäß einem oder mehreren der Ansprüche 1 bis 4, dadurch gekennzeichnet, daß die Polybase durch eine Derivatisierungsreaktion hydrophobisiert ist.
6. Polyelektrolytkomplexe gemäß einem oder mehreren der Ansprüche 1 bis 5, dadurch gekennzeichnet, daß die Polybase Polylysin, ein Lysinester, Chitosan oder Poly-(2-N,N-Dimethyl-aminoethyl)-D,L-aspartamid ist.
7. Polyelektrolytkomplexe, dadurch gekennzeichnet, daß sie aus Xylansulfat bzw. dessen lipophilen Derivaten und einer Polybase bestehen.
8. Polyelektrolytkomplexe, dadurch gekennzeichnet, daß sie aus Dextransulfat bzw. dessen lipophilen Derivaten und einer Polybase bestehen.
9. Polyelektrolytkomplexe, dadurch gekennzeichnet, daß sie aus Dextransulfat und Polylysin, einem Lysinester, Chitosan oder Poly(2-N,N-Dimethyl-aminoethyl)-D,L-aspartamid bestehen.
10. Verfahren zur Herstellung von Polyelektrolytkomplexen gemäß einem oder mehreren der Ansprüche 1 bis 9, dadurch gekennzeichnet, daß entweder a) die Polysäure oder b) die Polybase in wäßriger Lösung vorgelegt wird und a) die Polybase oder b) die Polysäure bei geeigneter Temperatur und bei geeignetem pH-Wert zugetropft wird.
11. Verwendung von Polyelektrolytkomplexen gemäß einem oder mehreren der Ansprüche 1 bis 9 zur Behandlung oder Prophylaxe von Viruserkrankungen.
12. Arzneimittel mit einem Gehalt an Polyelektrolykomplexen gemäß einem oder mehreren der Ansprüche 1 bis 9, gegebenenfalls neben üblichen Hilfs- und/oder Trägerstoffen.

Patentansprüche für folgende Vertragsstaaten:ES, GR

1. Verfahren zur Herstellung von Polyelektrolytkomplexen aus mindestens einer Polysäure und mindestens einer Polybase, dadurch gekennzeichnet, daß
   a) die Polysäure in wäßriger Lösung vorgelegt wird und daß die Polybase bei geeigneter Temperatur und bei geeignetem pH-Wert zugetropft wird, oder
   b) die Polybase in wäßriger Lösung vorgelegt wird und die Polysäure bei geeigneter Temperatur und bei geeignetem pH-Wert zugetropft wird.
2. Verfahren nach Anspruch 1, dadurch gekennzeichnet, daß die Polysäure ein teilweise oder vollständig sulfatiertes, unsubstituiertes oder substituiertes Oligo-oder Polysaccharid ist.
3. Verfahren nach Anspruch 1, dadurch gekennzeichnet, daß die Polysäure ein Oligo- oder Polysaccharid ist, deren OH-Gruppen teilweise verethert oder verestert sind.
4. Verfahren nach Anspruch 1, dadurch gekennzeichnet, daß die Polybase ein Protein ist.
5. Verfahren nach Anspruch 1, dadurch gekennzeichnet, daß die Polybase durch eine Derivatisierungsreaktion hydrophobisiert ist.
6. Verfahren nach Anspruch 1, dadurch gekennzeichnet, daß die Polybase Polylysin, ein Lysinester, Chitosan oder Poly-(2-N,N-Dimethyl-aminoethyl)-D,L-aspartamid ist.
7. Verfahren nach Anspruch 1, dadurch gekennzeichnet, daß Polyelektrolytkomplexe hergestellt werden, die aus Xylansulfat bzw. dessen lipophilen Derivaten und einer Polybase bestehen.
8. Verfahren nach Anspruch 1, dadurch gekennzeichnet, daß Polyelektrolytkomplexe hergestellt werden, die aus Dextransulfat bzw. dessen lipophilen Derivaten und einer Polybase bestehen.
9. Verfahren nach Anspruch 1, dadurch gekennzeichnet, daß sie aus Dextransulfat und Polylysin, einem Lysinester, Chitosan oder Poly(2-N,N-Dimethyl-aminoethyl)-D,L-aspartamid bestehen.

10. Verfahren zum Herstellen eines Medikaments zur Behandlung und Prophylaxe von Viruserkrankungen, dadurch gekennzeichnet, daß man eine wirksame Menge eines nach Anspruch 1 erhaltenen Polyelektrolytkomplexes mit den pharmazeutisch üblichen Hilfsstoffen in eine geeignete Darreichungsform bringt.